# NEUE EUROPÄISCHE PATENTSCHRIFT

(11) **EP 2 678 674 B2**
(45) Veröffentlichungstag und Bekanntmachung des Hinweises auf die Entscheidung über den Einspruch: **10.07.2019**
(45) Hinweis auf die Patenterteilung: 14.12.2016
(21) Anmeldenummer: 12704692.8
(22) Anmeldetag: 14.02.2012
(51) Int. Cl.: G01N 33/28, C10G 21/14, B01D 11/04, B01D 53/14

(54) **VERFAHREN ZUR BESTIMMUNG DES GEHALTS AN SCHWEFELWASSERSTOFF IN ROH- UND RÜCKSTANDSÖLEN**
METHOD FOR DETERMINING THE CONTENT OF HYDROGEN DISULFIDE IN CRUDE AND RESIDUAL OILS
PROCÉDÉ POUR DÉTERMINER LA TENEUR EN HYDROGÈNE SULFURÉ DANS DES HUILES BRUTES ET DES HUILES RÉSIDUELLES

(30) Priorität: 25.02.2011 DE 102011012445; 20.09.2011 DE 102011113943
(43) Veröffentlichungstag der Anmeldung: 01.01.2014
(73) Patentinhaber: Clariant International Ltd, 4132 Muttenz (CH)
(72) Erfinder: FEUSTEL, Michael, 55278 Köngernheim (DE); BRAUCHLE, Michael, 55543 Bad Kreuznach (DE); ANDRIN, Dominko, 65824 Schwalbach am Taunus (DE); KRULL, Matthias, 55296 Harxheim (DE)
(74) Vertreter: Mikulecky, Klaus
(86) Internationale Anmeldenummer: PCT/EP2012/000651
(87) Internationale Veröffentlichungsnummer: WO 2012/113519

(56) Entgegenhaltungen:
- US-A- 3 660 035
- US-A- 4 699 886
- US-A1- 2008 165 361
- ANONYMOUS: 'H2S Analyser with Vapour Phase Processor', [Online] 13 Mai 2015, Gefunden im Internet: <URL:http://seta-analytics.com/h2s-analyser .htm> [gefunden am 2015-07-20]
- M. Akstinat, S. Schaar, C. Marx: "Abschlussbericht zum Forschungsvorhaben "Herkunft, Bildung und Möglichkeiten zur Vermeidung der Entstehung von H2S beim Fluten von Erdöl-Lagerstätten mit überhitztem Wasserdampf"", 1979, Institut für Tiefbohrkunde und Erdölgewinnung, TU Clausthal * pages 11,15-18 *
- Arnold F Holleman, Egon Wiberg, Nils Wiberg: "Lehrbuch der anorganischen Chemie, 101. Auflage", 1995, Walter de Gruyter ISBN: 3-11-012641-9 * page 557 *
- Ingeborg Steinfatt: "Untersuchungen zur thermochemischen Bildung von H2S im Erdöl", 18 October 1993 (1993-10-18), TU Clausthal * pages 17,26,33 * * pages 39,45,51 *
- Hans Beyer, Wolfgang Walter: "Lehrbuch der Organischen Chemie, 21. Auflage", 1988, S. Hirzel Verlag, Stuttgart ISBN: 3-7776-0438-0 * page 148 *
- H.W. Prinzler: "Schwefel im Erdöl", 1968, VEB Deutscher Verlag für Grundstoffindustrie, Leibzig * page 30 *
- "Standard Test Method for Determination of Hydrogen Sulfide in Fuel Oils by Rapid Liquid Phase Extraction", ASTM D7621 - 10, 1 May 2010 (2010-05-01), pages 1-7, XP055421398,

## Beschreibung

Verfahren zur Bestimmung des Gehalts an Schwefelwasserstoff in Roh- und Rückstandsölen.

Die vorliegende Erfindung betrifft ein Verfahren zur Bestimmung des Gehalts an Schwefelwasserstoff von Roh- und Rückstandsölen sowie von Roh- und/oder Rückstandsöle enthaltenden Mineralöldestillaten.

Rohöle und die daraus in Raffinerien hergestellten Produkte enthalten üblicherweise größere Mengen an Schwefelwasserstoff (H₂S) und Mercaptanen, die sich beispielsweise bei der Lagerung dieser schwefelhaltigen Mineralöle im Gasraum über dem Öl anreichern können. Zum Beispiel bei Verladevorgängen kann dieses Gas freigesetzt werden und zu Geruchsbelästigung in der Umgebung sowie aufgrund seiner Toxizität auch zur Gefährdung von Mitarbeitern führen. Daher werden von der Legislative die in Mineralölprodukten zulässigen Gehalte an Schwefelwasserstoff immer weiter reguliert. So werden zukünftig gemäß ISO/FDIS 8217:2010(E) nur noch 2 ppm H₂S in Bunkerölen zulässig sein und auch für Rückstände der Mineralöldestillation wie beispielsweise Bitumen werden derartige Maßnahmen diskutiert. Weiterhin besitzt Schwefelwasserstoff insbesondere im Zusammenwirken mit Wasser stark korrosive Eigenschaften, was die Funktionalität und Sicherheit von Lager-, Transport- und Verarbeitungsanlagen beeinträchtigen kann. Folglich werden große Anstrengungen unternommen, den Gehalt von Roh- und Rückstandsölen wie auch von Mineralöldestillaten an Schwefelwasserstoff zu vermindern, um einerseits die Umweltbelastungen einzugrenzen und andererseits eine sichere Handhabung dieser Öle zu gewährleisten. Zur Bestimmung des H₂S-Gehalts in der Gasphase über schwefelhaltigen Mineralölen gibt es verschiedene etablierte Analyseverfahren. Dagegen bereitet die Bestimmung des in Roh- und Rückstandsölen sowie in Roh- und/oder Rückstandsölen enthaltenden Mineralöldestillaten gelösten bzw. gebundenen Schwefelwasserstoff- und Mercaptangehalts, der bei längerer Lagerung des Öls zusätzlich in die Gasphase übergehen kann, oftmals Schwierigkeiten, da eine direkte Analytik in diesen hoch viskosen und undurchsichtigen Ölen nicht möglich ist.

Ein Standard zur Bestimmung des Sulfidgehalts von flüssigen Mineralölprodukten einschließlich Destillationsrückstände enthaltenden Schiffstreibstoffen und Raffinerierohstoffen ist die IP 570. Dabei wird eine kleine Probemenge des zu untersuchenden Öls in einem Lösemittel verdünnt und unter definierten Bedingungen auf 60 °C erwärmt, um das darin gelöste H₂S freizusetzen. Mit einem durch die Probe geleiteten Luftstrom wird das H₂S zu einem Analysator geleitet, der die Konzentration über einen elektrochemischen Sensor misst. Weitere normierte Messverfahren sind beispielsweise UOP Methode 163-67 zur Bestimmung von Schwefelwasserstoff und Mercaptan-Schwefel in flüssigen Kohlenwasserstoffen mit Hilfe von potentiometrischer Titration und IP 399 zur Bestimmung von Schwefelwasserstoff in Rückstandölen mit Hilfe einer spektrophotometrischen Bestimmung. Auch diese Testmethoden werden üblicherweise bei 60 °C durchgeführt.

US 3,660,035 offenbart ein Verfahren, bei dem eine Probe in einer Mischung aus Toluol und Isoproylalkohol gelöst und anschließend mit CdCl₂-Lösung titriert wird. Insbesondere schwere und viskose Rohöle, Rückstandsöle und Roh- bzw. Rückstandsöle enthaltende Mineralöldestillate wie beispielsweise Mazut, schwere Heizöle und Bunkeröle werden oftmals bei erhöhten Temperaturen von 70 °C und mehr wie beispielsweise von 90 °C und mehr gelagert, um ihre Handhabbarkeit zu erleichtern. Bitumen wird oftmals sogar bei Temperaturen oberhalb 150 °C und teilweise oberhalb 180 °C gelagert. Dabei wird oftmals beobachtet, dass der nach bekannten Methoden in diesen Ölen bestimmte Gehalt an Schwefelwasserstoff schon nach kurzzeitiger Lagerung der Öle bei diesen erhöhten Temperaturen ansteigt. Ursache dafür dürften mit steigender Temperatur beschleunigte Reaktionen der im Öl enthaltenen Schwefelverbindungen wie beispielsweise zyklischen bzw. aromatischen Kohlenwasserstoffen mit gebundenem Schwefel, Mercaptanen und auch elementarem Schwefel zu H₂S sein. Dieses aus Ölbestandteilen beim Erwärmen nachgebildete H₂S wird im Rahmen dieser Erfindung als inhärentes H₂S bezeichnet. Somit kann man über die Bestimmung des H₂S-Gehalts gemäß bekannten Analysenverfahren wie beispielsweise der IP 570 nicht sicherstellen, dass der einmal gemessene und unterhalb der Grenzwerte liegende Gehalt an H₂S auch eine sichere Handhabung der Öle bei ihrer Erwärmung sowie bei ihrer weiteren Handhabung und Verarbeitung bei einer gegenüber den Messbedingungen der IP 570 erhöhten Temperatur gewährleistet. Oftmals ist der Gehalt an inhärentem H₂S zudem temperaturabhängig, so dass ein Öl bei unterschiedlichen Lagertemperaturen verschiedene Gehalte an H₂S enthalten kann. Hierbei ist auch zu berücksichtigen, dass Oberflächentemperaturen von Wärmetauschern und anderen Beheizvorrichtungen üblicherweise deutlich über den Temperaturen des eigentlichen Lagertanks liegen. Auch die Dauer der Warmlagerung der Öle oberhalb der Messtemperatur der IP 570 kann dieses Problem verschärfen.

Eine gebräuchliche Methode zur Absenkung des H₂S-Gehalts von Mineralölen ist deren Behandlung mit H₂S bindenden, als H₂S-Scavenger bezeichneten Zusätzen. Gebräuchliche H₂S-Scavenger sind beispielsweise Formaldehyd, dessen Umsetzungsprodukte mit Aminen wie beispielsweise Triazine, Glyoxal, Metalloxide, Metallsulfonate und andere Metallorganische Verbindungen. Üblicherweise ist dabei ein definiertes stöchiometrisches Vielfaches an H₂S-Scavenger pro Anteil an H₂S erforderlich. Bei der Behandlung von Roh- und Rückstandsölen sowie diese enthaltenden Mineralöldestillaten mit H₂S bindenden Zusätzen ist vor der Warmlagerung aus oben genannten Gründen eine zuverlässige Dosierung dieser Zusätze ohne prophylaktische, kostspielige Überadditivierung bisher nicht möglich, da keine Methode zur Bestimmung des Gehalts an inhärentem H₂S und somit keine Information über die Menge des sich bei der Lagerung dieser Öle bei erhöhten Temperaturen einstellenden H₂S zur Verfügung steht. Dabei wäre ein Messverfahren wünschenswert, das einerseits die Bestimmung niedriger H₂S-Konzentrationen von weniger als 10 ppm und insbesondere weniger als 2 ppm beispielsweise für den Nachweis eingehaltener Spezifikationen ermöglicht und andererseits auch die Bestimmung höherer H₂S-Gehalte von mehr 10 ppm und insbesondere mehr als 25 ppm erlaubt, um eine gezielte Dosierung von Scavengern vornehmen zu können.

Es bestand folglich die Aufgabe, ein Verfahren zur Bestimmung des Gehalts von schwefelhaltigen Roh- und Rückstandsölen sowie schwefelhaltige Roh- und/oder Rückstandsöle enthaltenden Mineralöldestillaten (im Rahmen dieser Anmeldung auch gemeinsam als schwefelhaltige Mineralöle bezeichnet) an gelöstem und inhärentem H₂S bereitzustellen. Insbesondere soll eine Bestimmung des sich im Laufe der Lagerung des schwefelhaltigen Mineralöls bei erhöhten Temperaturen einstellenden inhärenten H₂S-Gehalts ermöglicht werden. Dies würde wiederum eine zuverlässige Bestimmung der für eine dauerhafte Absenkung des H₂S-Gehalts erforderlichen Dosierrate an H₂S-Scavenger erlauben. Weiterhin soll das Verfahren wenig Zeitbedarf erfordern.

Überraschenderweise wurde gefunden, dass die Bestimmung des sich während der Lagerung schwefelhaltiger Roh- und Rückstandsöle sowie schwefelhaltige Roh- und/oder Rückstandsöle enthaltender Mineralöldestillate bei Temperaturen oberhalb 80 °C wie beispielsweise bei Temperaturen zwischen 100 bis 280 °C einstellenden H₂S-Gehalts dadurch möglich ist, dass das zu analysierende schwefelhaltige Mineralöl in einem hochsiedenden Lösemittel gelöst wird und diese Lösung bei Temperaturen oberhalb 80 °C und insbesondere bei der relevanten Lagertemperatur mit einem Trägergas durchspült und die mit dem Trägergas aus dem schwefelhaltigen Mineralöl ausgetragene Menge H₂S quantifiziert wird.

Gegenstand der Erfindung ist dementsprechend ein Verfahren zur Bestimmung des sich bei Warmlagerung von schwefelhaltigen Roh- und Rückstandsölen sowie schwefelhaltige Roh- und/oder Rückstandsöle enthaltenden Mineralöldestillaten einstellenden H₂S-Gehalts, in dem eine Probe des schwefelhaltigen Mineralöls in einem oberhalb 200 °C siedenden Lösemittel oder Lösemittelgemisch gelöst wird und die Lösung des schwefelhaltigen Mineralöls bei Temperaturen oberhalb 80 °C mit einem Trägergas durchströmt wird und die mit dem Trägergas ausgetragene Menge an Schwefelwasserstoff quantitativ analysiert wird.

Ein weiterer Gegenstand der Erfindung ist ein Verfahren zur Bestimmung der Konzentrationen von gelöstem Schwefelwasserstoff und inhärentem Schwefelwasserstoff in schwefelhaltigen Roh- und Rückstandsölen sowie schwefelhaltige Roh- und/oder Rückstandsöle enthaltenden Mineralöldestillaten, in dem eine Probe des schwefelhaltigen Mineralöls in einem oberhalb 200 °C siedenden Lösemittel oder Lösemittelgemisch gelöst wird und die Lösung des schwefelhaltigen Mineralöls zuerst zur Bestimmung des Gehalts an gelöstem Schwefelwasserstoff bei einer Temperatur unterhalb 100 °C mit einem Trägergas durchströmt wird und die mit dem Trägergas ausgetragene Menge an Schwefelwasserstoff quantitativ analysiert wird und anschließend die derart behandelte Probe zur Bestimmung des inhärenten Schwefelwasserstoffs unter weiterem Spülen mit Trägergas auf Temperaturen oberhalb 100 °C erhitzt und das mit dem Trägergas ausgetragene H₂S quantitativ analysiert wird, wobei die Temperatur bei der Bestimmung des inhärenten H₂S höher liegt als die Temperatur bei der Bestimmung des gelösten H₂S.

Ein weiterer Gegenstand der Erfindung ist ein Verfahren zur Bestimmung der zum Binden von H₂S bei Lagerung von schwefelhaltigen Roh- und Rückstandsölen sowie schwefelhaltige Roh- und/oder Rückstandsöle enthaltenden Mineralöldestillaten erforderlichen Konzentration an H₂S-Scavengern, indem eine Probe des schwefelhaltigen Mineralöls in einem oberhalb 200 °C siedenden Lösemittel oder Lösemittelgemisch gelöst wird und die Lösung des schwefelhaltigen Mineralöls bei Temperaturen oberhalb 80 °C mit einem Trägergas durchströmt wird und die mit dem Trägergas ausgetragene Menge an gelöstem und inhärentem Schwefelwasserstoff quantitativ analysiert und daraus die für dauerhafte Absenkung des H₂S-Gehalts erforderliche Menge an H₂S-Scavenger berechnet wird.

Das Verfahren eignet sich für die Bestimmung eines weiten Konzentrationsbereichs an H₂S. Bevorzugt eignet es sich für die Bestimmung von H₂S im Bereich von 0,01 bis 5.000 ppm, besonders bevorzugt im Bereich von 0,1 bis 1.000 ppm und speziell im Bereich von 0,2 bis 100 ppm wie beispielsweise im Bereich von 0,5 bis 50 ppm H₂S, jeweils bezogen auf die Menge an gelöstem beziehungsweise inhärentem Schwefelwasserstoff.

In einer bevorzugten Ausführungsform des erfindungsgemäßen Verfahrens lassen sich die Konzentrationen an gelöstem und inhärentem H₂S nebeneinander bestimmen. Dazu wird im ersten Schritt der Gehalt des schwefelhaltigen Roh- und Rückstandsöls bzw. des schwefelhaltiges Roh- und/oder Rückstandsöl enthaltenden Mineralöldestillats an gelöstem H₂S durch Spülen mit Trägergas bei einer relativ niedrigen Temperatur von unterhalb 100 °C, bevorzugt zwischen 60 °C und 90 °C wie beispielsweise bei 80, 75, 70, 65 oder 60 °C ausgetragen und quantitativ analysiert. Anschließend wird diese Probe zur Bestimmung des inhärenten H₂S unter weiterem Spülen mit Trägergas auf Temperaturen oberhalb 100 °C und insbesondere oberhalb 120 °C erhitzt und das mit dem Trägergas ausgetragene H₂S quantitativ analysiert. Die Temperatur zur Bestimmung des inhärenten H₂S liegt dabei bevorzugt mindestens 10 °C und insbesondere mindestens 20 °C höher als die Temperatur zur Bestimmung des gelösten H₂S.

In einer weiteren bevorzugten Ausführungsform des erfindungsgemäßen Verfahrens lässt sich der Gesamtgehalt von schwefelhaltigen Roh- und Rückstandsölen bzw. schwefelhaltigen Roh- und/oder Rückstandsöl enthaltenden Mineralöldestillaten an gelöstem und inhärentem H₂S bestimmen. Dazu wird das in einem Lösemittel oder Lösemittelgemisch gelöste schwefelhaltige Mineralöl bei Temperaturen oberhalb 100 °C, bevorzugt zwischen 120 und 300 °C wie beispielsweise zwischen 130 und 250 °C mit einem Trägergas durchströmt und die mit dem Trägergas ausgetragene Menge an gelöstem und inhärentem Schwefelwasserstoff quantitativ analysiert.

Bevorzugt wird die Bestimmung des Gehalts an inhärentem H₂S bei einer Temperatur vorgenommen, die mindestens der Temperatur entspricht, der das schwefelhaltige Roh- oder Rückstandsöl bzw. der das schwefelhaltige Roh- und/oder Rückstandsöl enthaltende Mineralöldestillat beispielsweise bei Transport oder Lagerung ausgesetzt wird. Besonders bevorzugt wird sie bei einer Temperatur vorgenommen, die mindestens 5 °C, insbesondere mindestens 10 °C und speziell mindestens 20 °C oberhalb der zu prüfenden Temperatur liegt. Bei der Betrachtung des "worst case" haben sich auch Temperaturen von 30 oder 50 °C oberhalb der im regulären Betrieb zu erwartenden Temperaturen bewährt. An schwefelhaltigen Roh- und/oder Rückstandsöl enthaltenden Mineralöldestillaten wird das erfindungsgemäße Verfahren bevorzugt unterhalb des Siedebeginns des Mineralöldestillats wie beispielsweise mindestens 10 °C und bevorzugt mindestens 20 °C unterhalb des Siedebeginns des Mineralöldestillats durchgeführt.

Als Trägergas bevorzugt sind unter den verfahrensgemäßen Temperaturbedingungen gegenüber dem zu analysierenden Öl chemisch inerte Gase und Sauerstoff. Als chemisch inerte Gase sind Stickstoff, Kohlendioxid, Edelgase wie beispielsweise Helium, Argon und deren Mischungen bevorzugt. Besonders bevorzugtes inertes Gas ist Stickstoff. Üblicherweise werden als Trägergase technische Qualitäten mit einer Reinheit von mindestens 99 Vol.-% und insbesondere mit mindestens 99,9 Vol.-% eingesetzt. Für den Fall, dass der zur quantitativen Bestimmung des ausgetragenen H₂S eingesetzte Analysator eine Mindestmenge an Sauerstoff im Trägergas benötigt, kann dem Trägergas vor oder nach dem Durchströmen der zu analysierenden Öllösung eine entsprechende Sauerstoffmenge zugesetzt werden. Bevorzugt wird er dem Trägergas nach dem Durchströmen der zu analysierenden Lösung des Öls zugesetzt. In einer besonders bevorzugten Ausführungsform wird Luft mit ihrem natürlichen Gehalt an Sauerstoff als Trägergas eingesetzt. In einer weiteren bevorzugten Ausführungsform wird mit Sauerstoff angereicherte Luft eingesetzt. In einer weiteren bevorzugten Ausführungsform wird reiner Sauerstoff mit einer Reinheit von ≥ 99,5 Vol.-% (O₂ technisch) eingesetzt.

Bevorzugt wird das erfindungsgemäße Verfahren bei Atmosphärendruck durchgeführt. In speziellen Fällen wie beispielsweise bei der Bestimmung des Gehalts an inhärentem H₂S von Ölen mit niedriger siedenden Bestandteilen kann es auch unter Überdruck wie beispielsweise bei 1,01 bis 50 bar (absolut) durchgeführt werden.

Das Trägergas kann mittels eines Rohres in die Lösung des zu untersuchenden Öls eingeleitet werden. Bevorzugt erfolgt das Einleiten über eine Fritte, wodurch das Trägergas besser verteilt wird und die Durchführung des Verfahrens deutlich beschleunigt wird. Bevorzugt befindet sich die Fritte möglichst dicht über dem Boden des Reaktionsgefäßes und speziell taucht sie vollständig in die Lösung des zu analysierenden Öls ein. Bevorzugte Fritten sind aus Edelstahl, Keramik oder Glas. Bevorzugt beträgt die Nennweite ihrer Poren zwischen 10 und 500 µm und besonders bevorzugt zwischen 50 und 300 µm. So haben sich beispielsweise Glas-, Keramik- und Metallfritten der Kennzeichnungen POR-00 / G 00, POR-0 / G 0 und POR-1 / G 1 besonders bewährt.

Das Durchströmen der Öllösung mit Trägergas ist so lange fortzusetzen, bis die Konzentration des vom Analysator detektierten H₂S auf den Basiswert zurückgegangen ist. Die Dauer ist somit abhängig von der H₂S-Konzentration in der zu analysierenden Probe, der Messtemperatur und der chemischen Natur der in der Probe enthaltenen, zu H₂S zerfallenden Schwefelverbindungen.

Für die Durchführung des erfindungsgemäßen Verfahrens bevorzugte Lösemittel und Lösemittelgemische haben einen Siedepunkt bei Temperaturen oberhalb 250 °C und insbesondere oberhalb 300 °C. bzw. Im Falle von Lösemittelgemischen stehen die Temperaturangaben zum Siedepunkt für den Siedebeginn des Lösemittelgemischs. Weiterhin liegt ihre Zersetzungstemperatur bevorzugt oberhalb 260 °C und insbesondere oberhalb 280 °C wie beispielsweise oberhalb 320 °C. Bevorzugte Lösemittel können aromatisch oder aliphatisch sein. Bevorzugt sind sie aliphatisch oder zumindest überwiegend aliphatisch, das heißt sie enthalten bevorzugt mindestens 80 Gew.-%, besonders bevorzugt mindestens 90 Gew.-% und insbesondere mindestens 92 Gew.-% aliphatischer Verbindungen und bevorzugt weniger als 20 Gew.-%, besonders bevorzugt weniger als 10 Gew.-% und insbesondere weniger als 8 Gew.-% an aromatischen Verbindungen. Bevorzugte aliphatische Lösemittel sind paraffinischer oder naphthenischer Natur oder Mischungen aus paraffinischen und naphthenischen Ölen im Verhältnis 1:50 bis 50:1. Besonders bevorzugte Lösemittel sind weitestgehend gesättigt. Weitestgehend gesättigt bedeutet, dass ihre nach Kaufmann bestimmte Jodzahl bevorzugt unter 20 g I₂/100 g und insbesondere unter 10 g I₂/100 g wie beispielsweise unterhalb 5 g I₂/100 g beträgt. Bevorzugte Lösemittel sind im Wesentlichen schwefelfrei, das heißt ihr Schwefelgehalt liegt bevorzugt unter 1.000 ppm (w/w), besonders bevorzugt unter 500 ppm (w/w) und insbesondere unter 100 ppm wie beispielsweise unter 10 ppm (w/w). Beispiele für geeignete Lösemittel sind Grundöle der Gruppen I, II und III sowie Poly(α-olefine).

Die Durchführung des erfindungsgemäßen Verfahrens erfolgt bevorzugt in einer Vorrichtung, die ein beheizbares Reaktionsgefäß mit Rührer, Thermometer, Probeneinlass, Gaseinleitung für das Trägergas und eine zu einem Analysator führende Gasableitung enthält. Das zu analysierende schwefelhaltige Roh- oder Rückstandsöl bzw. das schwefelhaltige Roh- oder Rückstandsöl enthaltende Mineralöldestillat wird in dem beheizbaren Reaktionsgefäß im hochsiedenden Lösemittel gelöst. In einer bevorzugten Ausführungsform wird die Lösung des zu untersuchenden Öls im Lösungsmittel in das Reaktionsgefäß gefüllt und dann unter Durchleiten von Trägergas erhitzt. In einer besonders bevorzugten Ausführungsform wird das im Reaktionsgefäß vorgelegte Lösemittel auf Analysentemperatur erhitzt und das zu analysierende schwefelhaltige Mineralöl über einen bevorzugt gasdicht ausgelegten Probeneinlass zudosiert. Besonders bewährt haben sich dabei Volumina zwischen 10 und 500 ml und speziell 50 bis 200 ml Lösemittel. Die einzusetzende Menge des zu analysierenden schwefelhaltigen Mineralöls hängt stark von dessen Gehalt an H₂S und/oder der Art des verwendeten Analysators ab. Sie liegt üblicherweise zwischen 0,001 g und 10 g und bevorzugt zwischen 0,01 g und 1 g. Die Konzentration des zu analysierenden Öls im Lösemittel liegt bevorzugt zwischen 0,1 und 5 Gew.-%, besonders bevorzugt zwischen 0,1 und 1 Gew.-%.

Das Reaktionsgefäß ist bevorzugt aus chemisch inertem Material wie Glas, Keramik oder Edelstahl gefertigt. Es ist mit einem Rühraggregat, einem Thermometer zur Kontrolle der Temperatur, einer Gaseinleitung unter die Flüssigkeitsoberfläche sowie einer Gasableitung aus dem Gasraum ausgestattet. Die Gaseinleitung ist bevorzugt mit Ventil und Durchflussmesser zur Regulierung und Messung des Trägergasstroms versehen. Bevorzugt werden Volumenströme an Trägergas von 5 bis 200 Liter pro Stunde, besonders bevorzugt von 10 bis 100 Liter pro Stunde wie beispielsweise 20 bis 70 Liter pro Stunde verwendet. Über die Gasableitung wird das Trägergas zu einem Analysator geleitet. Oftmals hat es sich dabei bewährt, das Trägergas vor dem Eintritt in den Analysator einer Reinigung zu unterziehen, um beispielsweise mitgerissene Lösemitteldämpfe oder leichter siedende Bestandteile des zu analysierenden Öls, die gegebenenfalls zu einer Verfälschung der Messergebnisse führen können, aus dem Trägergasstrom abzutrennen. Dies kann beispielsweise mittels einer Kühlfalle erfolgen.

Zur Detektion des Schwefelwasserstoffs im Luftstrom sind prinzipiell alle bekannten Methoden zur quantitativen Bestimmung von H₂S geeignet. Beispielsweise kann sie mit Gasdetektor-Röhrchen bestimmt werden, bei denen das Trägergas über eine körnige Reaktionsschicht geleitet wird und beispielsweise die Länge der angefärbten Indikatorzone oder ein Farbintensitätsvergleich Auskunft über die Gaskonzentration gibt. Geeignete Absorptionsröhrchen sind beispielsweise unter der Bezeichnung Dräger-Röhrchen^{®} der Firma Drägerwerk AG & Co. KGaA verfügbar. Bevorzugt erfolgt die Detektion mit elektrochemischen Sensoren. Diese ermitteln und registrieren die H₂S-Konzentration im Luftstrom zu jedem Zeitpunkt des Verfahrens. Beispielsweise geeignete Analysatoren sind Gasmessgeräte vom Typ S4000T der Firma General Monitors, der Typen PT 205, PT295 oder PT395 der Firma PemTech Inc., des Typs TGS 825 der Firma Figaro Engineering Inc. (Japan) sowie Typ Dräger X-am^{®} 5000 der Firma Drägerwerk AG & Co. KGaA. H₂S-spezifische Sensoren sind beispielsweise Dräger Sensor XXS H2S LC - 68 11 525, Dräger Sensor XXS H2S - 68 10 883 und Dräger Sensor XXS H2S HC - 68 10 883. Nach vollständigem Austragen des Schwefelwasserstoffs aus dem Reaktionskolben, was durch Absinken des H₂S-Gehalts auf die Basislinie des Sensors erkennbar ist, wird der H₂S-Gehalt durch Integration über die Zeit ermittelt.

Bei der Verwendung von Sauerstoff oder sauerstoffhaltigen Gasen als Trägergas wird oftmals eine zumindest partielle Oxidation des Schwefelwasserstoffs zu Schwefeldioxid (SO₂) beobachtet. Um eine dadurch verursachte Verfälschung der Messwerte zu vermeiden hat es sich oftmals bewährt, auch den Gehalt von SO₂ im Trägergas quantitativ zu bestimmen und ihn nach Umrechnung auf H₂S letzterem hinzuzurechnen. Die Bestimmung von SO₂ kann nach bekannten Methoden wie beispielsweise mit spezifischen Gasdetektor-Röhrchen oder elektrochemischen Sensoren erfolgen. Bei dieser Ausführungsform ist sicherzustellen, dass das zu untersuchende schwefelhaltige Mineralöl selbst kein SO₂ enthält bzw. dass eventuell im schwefelhaltigen Mineralöl enthaltenes SO₂ bei der Berechnung des H₂S-Gehalts berücksichtigt wird.

Das erfindungsgemäße Verfahren ist allgemein für die Bestimmung des in schwefelhaltigen Mineralölen enthaltenen sowie des sich bei erhöhten Temperaturen einstellenden Gehalts an H₂S in Rohölen, Rückstandsölen und diese Roh- und/oder Rückstandsöle enthaltenden Mineralöldestillaten geeignet. Unter Rückstandsölen werden Rückstände der Mineralöldestillation verstanden, die als nicht mehr verdampfbarer Teil eines erdölverarbeitenden (meist destillativen) Prozesses entstanden sind. Sie können beispielsweise bei der Mineralöldestillation bei Normaldruck oder im Vakuum wie auch als Rückstände in Konversionsanlagen wie beispielsweise als Rückstände in Visbreakern oder Crackern angefallen sein. Besonders geeignet ist das Verfahren für die Bestimmung des Gehalts an H₂S in Bitumen und Asphalt wie beispielsweise in Destillationsbitumen, Fluxbitumen, Hartbitumen, Hochvakuumbitumen und Oxidationsbitumen. Auch für die Bestimmung des Schwefelwasserstoffs in polymermodifiziertem Bitumen ist das Verfahren geeignet.

Oftmals werden Rückstände der Mineralöldestillation durch Blenden mit anderen Raffinerieprodukten zu Endprodukten verarbeitet. Bevorzugte zum Blenden oder Lösen bzw. Verdünnen der Roh- bzw. Rückstandsöle eingesetzte Mineralöldestillate sind hochsiedende Fraktionen der Mineralöldestillation und insbesondere Destillate aus der Vakuumdestillation sowie aus Crack- und anderen Konversionsanlagen. Bevorzugte Mineralöldestillate haben Siedepunkte oberhalb 250 °C und insbesondere oberhalb 300 °C. Oftmals haben diese Mineralöldestillate einen vergleichsweise hohen Schwefelgehalt von mehr als 100 ppm, wie beispielsweise zwischen 200 und 10.0000 ppm. Bevorzugt eingesetzte Mineralöldestillate sind Vakuumgasöl (VGO, HVGO), Light Cycle Oil (LCO), Heavy Cycle Oil (HCO), Visbreaker Gasöl bzw. Visbreaker-Vakuumdestillat (Flashed Cracked Distillate) oder Slurry aus der FCC-Anlage. Das Mischungsverhältnis zwischen Roh- bzw. Rückstandsöl und Mineralöldestillat wird üblicherweise so eingestellt, dass die Viskosität der Mischung der angestrebten Viskosität entspricht. Bevorzugt liegt das Mischungsverhältnis zwischen 20:1 und 1:20, bevorzugt 10:1 und 1:10 (Masse/Masse). Beispiele für derartige Rückstandsöle enthaltende Mineralöldestillate sind Bunkeröle und Heizöle wie insbesondere schwere Heizöle.

Insbesondere ist das erfindungsgemäße Verfahren für die Bestimmung des sich bei erhöhten Temperaturen einstellenden Gehalts an H₂S in Bunkerölen, schweres Heizöl (Schweröl), Bitumen und Asphalt geeignet.

Das erfindungsgemäße Verfahren ermöglicht neben der Bestimmung von gelöstem Schwefelwasserstoff auch die Bestimmung von Schwefelwasserstoff, der während einer Lagerung des Öls bei erhöhten Temperaturen aus Schwefel enthaltenden Verbindungen gebildet wird (inhärentes H₂S). Darüber hinaus lässt sich die Konzentration an inhärentem H₂S in Abhängigkeit von der Temperatur ermitteln. Damit ist auch eine Aussage über die sichere Handhabbarkeit derartiger Öle nach längerer Lagerung auch bei verschiedenen erhöhten Temperaturen möglich. Die Verwendung eines hoch siedenden Lösemittels erlaubt eine lange Verwendbarkeit herkömmlicher, auf Lösemitteldämpfe empfindlich reagierender elektrochemischer Sensoren.

Dabei ermöglicht das erfindungsgemäße Verfahren einerseits die Bestimmung niedriger H₂S-Konzentrationen von weniger als 10 ppm und insbesondere weniger als 2 ppm wie es beispielsweise für den Nachweis einzuhaltender Spezifikationen erforderlich ist. Andererseits erlaubt es auch die Bestimmung höherer H₂S-Gehalte von mehr 10 ppm und insbesondere mehr als 25 ppm wie beispielsweise mehr als 50 ppm, um eine gezielte Dosierung von H₂S-Scavengern vornehmen zu können. Dabei eignet es sich sowohl zur Simulierung des bei Produktions- und/oder Lagerbedingungen des schwefelhaltigen Roh- oder Rückstandsöls zu erwartenden H₂S-Gehalts wie auch zur Bestimmung des bei längerer Lagerung des Öls unter gegebenen Bedingungen zu erwartenden H₂S-Gehalts. Weiterhin kann es zur Evaluierung geeigneter Additive zur Absenkung des H₂S-Gehalts (H₂S-Scavenger) wie auch zur Bestimmung der zur Einstellung eines gewünschten H₂S-Gehalts erforderlichen Dosierrate des Additivs eingesetzt werden. Damit ist die Bestimmung einer auch für längere Lagerung bei erhöhten Temperaturen ausreichende Dosierrate an Scavenger möglich, ohne dass es zu prophylaktischer Überadditivierung und damit unnötigen Kosten kommt.

### Beispiele

### Bestimmung des H₂S-Gehalts an schweren Rückstandsölen und Bitumen

Zur Bestimmung des Schwefelwasserstoffgehalts von Roh- und Rückstandsölen wurden in einem 100 ml-Gefäß mit Magnetrührer, Innenthermometer und Gasableitung 50 ml Lösemittel vorgelegt und mit 0,1 bis 1 g des zu analysierenden Roh- oder Rückstandsöls versetzt. Das verwendete Lösemittel bestand vorwiegend aus Aliphaten (>90 %) und hatte einen Siedebeginn oberhalb 250 °C. Unter Durchleiten eines Luft- bzw. Stickstoffstroms (Trägergasstrom; 20 l/h), der über eine G2-Fritte aus Edelstahl unter die Oberfläche des Lösemittels appliziert wurde, wurde das Öl auf die angegebene Temperatur T₁ und nachdem der H₂S-Gehalt im Trägergasstrom auf den Ausgangswert (∼0 ppm) gefallen war, weiter auf T₂ erhitzt.

Der Luft- bzw. Stickstoffstrom (Trägergasstrom) wurde über die Gasableitung zu einem elektrochemischen, spezifisch auf H₂S reagierenden Gassensor geleitet und der H₂S-Gehalt zeitabhängig registriert. Nach insgesamt etwa 15 bis 30 Minuten war der H₂S-Gehalt im Luft- bzw. Stickstoffstrom wieder auf den Ausgangswert abgesunken, woraufhin die Messung beendet und der H₂S-Gehalt des Öls durch Integration über die Zeit berechnet wurde. Durch Integration des Signals bei T₁ wurde der Gehalt an gelöstem H₂S und durch Integration es Signals bei T₂ der Gehalt an inhärentem H₂S bestimmt. Die Summe aus gelöstem und inhärentem H₂S entspricht dem Gesamtgehalt an H₂S. Bei den angegebenen Messwerten handelt es sich jeweils um den Mittelwert aus drei Messungen.

### Beispiel 1

Bei verschiedenen Temperaturen wurde in einem Heavy Fuel Oil (mit VGO geblendeter Rückstand der Vakuumdestillation; HFO I) bzw. in einem Bitumen (Rückstand aus Visbreaker bei Verarbeitung von Rohöl aus der Provenienz Mittlerer Osten; Bitumen I) der Gehalt an Schwefelwasserstoff bestimmt. Durch Messung bei 60 °C (T₁) wurde dabei der Gehalt an gelöstem H₂S und bei höherer Temperatur (T₂) der sich bei dieser Temperatur einstellende Gesamtgehalt an H₂S bestimmt. Als Trägergas wurde Luft verwendet. Bei den Messungen 3, 7 und 9 wurden die Proben nach der Messung bei 60 °C auf die in Tabelle 1 angegebene Temperatur T₂ erhitzt und die sich bei dieser erhöhten Temperatur einstellende Konzentration an inhärentem H₂S bestimmt. Die Gesamtmenge an gelöstem und inhärentem H₂S entspricht bei diesen Messungen der in einem Schritt bestimmten Gesamtkonzentration.

**Tabelle 1: Bestimmung des H₂S-Gehalts in Abhängigkeit von der Temperatur**

| Messung | Öl | T₁ [°C] | H₂S (T₁) [ppm] | T₂ [°C] | H₂S (T₂) [ppm] |
|---|---|---|---|---|---|
| 1 | HFO I | - | - | 120 | 29 |

| Messung | Öl | T₁ [°G] | H₂S (T₁) [ppm] | T₂ [°G] | H₂S (T₂) [ppm] |
|---|---|---|---|---|---|
| 2 | HFO I | - | - | 150 | 37 |
| 3 | HFO I | 60 | 25 | 150 | 13 |
| 4 | Bitumen I | - | - | 140 | 38 |
| 5 | Bitumen I | - | - | 160 | 41 |
| 6 | Bitumen I | - | - | 180 | 54 |
| 7 | Bitumen I | 60 | 35 | 180 | 18 |
| 8 | Bitumen I | - | - | 200 | 68 |
| 9 | Bitumen I | 60 | 36 | 200 | 35 |

### Beispiel 2

Zur Absenkung des Gehalts an Schwefelwasserstoff wurden ein Heavy Fuel Oil (mit LCO und FCC-slurry geblendeter Rückstand aus Visbreaker; HFO II) sowie ein Bitumen (Rückstand aus Visbreaker bei Verarbeitung von Rohöl südamerikanischer Provenienz; Bitumen II) mit H₂S-Scavenger versetzt. Bei den eingesetzten H₂S-Scavengern handelte es sich um kommerziell verfügbare Produkte auf Basis eines Reaktionsprodukts aus Aldehyd und Amin (Scavenger A) bzw. auf Basis einer metallorganischen Verbindung (Scavenger B). Für beide Produkte wird eine Dosierung von 5 Gewichtsteilen Scavenger pro Gewichtsteil H₂S empfohlen. Die Dosierung erfolgte zum einen bezogen auf den Gehalt an gelöstem H₂S (Messungen 11 und 14) und zum anderen bezogen auf die Gesamtkonzentration an gelöstem und inhärentem H₂S (Messungen 12 und 15). Zur Bestimmung von gelöstem und inhärentem H₂S wurden die Öle wiederum stufenweise auf die angegebenen Temperaturen T₁ und T₂ erhitzt. Die angegebenen Dosierraten beziehen sich jeweils auf die Menge an eingesetztem Wirkstoff (Gew./Gew.). Als Trägergas wurde Luft verwendet.

**Tabelle 2: Bestimmung des Gehalts an freiem und gelöstem H₂S in Heavy Fuel Oil (HFO II) vor und nach Zugabe von Scavenger**

| Messung | Scavenger A | T₁ | H₂S (T₁) | T₂ | H₂S (T₂) | H₂S gesamt |
|---|---|---|---|---|---|---|
| | [ppm] | [°C] | [ppm] | [°C] | [ppm] | [ppm] |
| 10 | - | 60 | 82 | 150 | 20 | 102 |
| 11 | 410 | 60 | 1,5 | 150 | 18 | 19,5 |
| 12 | 500 | 60 | 0,5 | 150 | 1,2 | 1,7 |

**Tabelle 3: Bestimmung des Gehalts an freiem und gelöstem H₂S in Bitumen II vor und nach Zugabe von Scavenger**

| Messung | Scavenger B | T₁ | H₂S (T₁) | T₂ | H₂S (T₂) | H₂S gesamt |
|---|---|---|---|---|---|---|
| | [ppm] | [°C] | [ppm] | [°C] | [ppm] | [ppm] |
| 13 | - | 60 | 30 | 180 | 13 | 43 |
| 14 | 150 | 60 | 4,4 | 180 | 12 | 16,4 |
| 15 | 215 | 60 | 1,8 | 180 | 2,3 | 4,1 |

### Beispiel 3

In Analogie zu Beispiel 2 wurde ein Bitumen (Rückstand aus der Vakuumdestillation von Arab Heavy-Rohöl; Bitumen III) mit H₂S-Scavenger A gemäß Beispiel 2 versetzt. Die Dosierung erfolgte wiederum mit 5 Gewichtsteilen Scavenger pro Gewichtsteil H₂S zum einen bezogen auf den Gehalt an gelöstem H₂S (Messung 17) und zum anderen bezogen auf die Gesamtkonzentration an gelöstem und inhärentem H₂S (Messung 18). Die angegebenen Dosierraten beziehen sich jeweils auf die Menge an eingesetztem Wirkstoff (Gew./Gew.). Als Trägergas wurde Stickstoff verwendet.

**Tabelle 4: Bestimmung des Gehalts an freiem und gelöstem H₂S in Bitumen III vor und nach Zugabe von Scavenger**

| Messung | Scavenger A | T₁ | H₂S(T₁) | T₂ | H₂S (T₂) | H₂S gesamt |
|---|---|---|---|---|---|---|
| | [ppm] | [°C] | [ppm] | [°C] | [ppm] | [ppm] |
| 16 | - | 60 | 18 | 170 | 8 | 26 |
| 17 | 72 | 60 | 1,9 | 170 | 6 | 7,9 |
| 18 | 104 | 60 | 0,9 | 170 | 0,7 | 1,6 |

### Beispiel 4

In Analogie zu Beispiel 2 wurde unter Durchleiten eines Luftstroms (10 l/h) ein Bitumen (Rückstand aus der Vakuumdestillation von Kuwait-Rohöl; Bitumen IV) vor und nach Zugabe von H₂S-Scavenger A stufenweise auf die angegebenen Temperaturen T₁ und T₂ erhitzt. Abweichend vom Verfahren des Beispiels 2 wurden in der Gasableitung sowohl der H₂S- wie auch der SO₂-Gehalt mit spezifischen Sensoren zeitabhängig registriert. Nach etwa 20 Minuten waren der H₂S- und der SO₂-Gehalt im Trägergas wieder auf den Ausgangswert abgesunken, woraufhin die Messung beendet und H₂S- wie auch SO₂-Gehalt des Öls durch Integration über die Zeit berechnet wurden (Messung 19). Durch Umrechnung des in SO₂ enthaltenen Schwefels in H₂S wurde die ursprünglich im Bitumen enthaltene Menge an gelöstem H₂S (T₁, gesamt) und inhärentem H₂S (T₂, gesamt) bestimmt. Durch Addition von gelöstem und inhärentem H₂S berechnet sich der Gesamtgehalt an H₂S.

Die Dosierung des H₂S-Scavengers A erfolgte zum einen bezogen auf den Gesamtgehalt an gelöstem H₂S (T₁, gesamt; Messung 20) und zum anderen bezogen auf die Gesamtkonzentration an gelöstem und inhärentem H₂S (Messung 21). Die angegebenen Dosierraten beziehen sich jeweils auf die Menge an eingesetztem Wirkstoff (Gew./Gew.).

**Tabelle 5: Bestimmung des Gehalts an freiem und gelöstem H₂S in Bitumen IV vor und nach Zugabe von Scavenger**

| Messung | Scavenger A | T₁ | H₂S (T₁) | SO₂ (T₁) | H₂S (T₁, gesamt) | T₂ [°C] | H₂S (T₂) | SO₂ (T₂) | H₂S (T₂, gesamt) | H₂S gesamt |
|---|---|---|---|---|---|---|---|---|---|---|
| | [ppm] | [°C] | [ppm] | [ppm] | [ppm] | | [ppm] | [ppm] | [ppm] | [ppm] |
| 19 | - | 60 | 45 | 10 | 50 | 190 | 13 | 4 | 15 | 65 |
| 20 | 250 | 60 | 6,3 | 1,8 | 7,3 | 190 | 10 | 4 | 12 | 19,3 |
| 21 | 325 | 60 | 0,5 | 0,3 | 0,6 | 190 | 0,6 | 0,4 | 0,8 | 1,4 |

Die Versuche zeigen, dass das erfindungsgemäße Verfahren eine Bestimmung des gelösten wie auch des sich bei verschiedenen Temperaturen einstellenden inhärenten Schwefelwasserstoffs ermöglicht. Damit erlaubt es auch die Abschätzung des sich bei der Lagerung bei erhöhter Temperatur einstellenden Gehalts an inhärentem H₂S. Durch sequentielle Messung der H₂S-Gehalte bei Temperaturen um 60 °C und höheren Temperaturen können sowohl der Gehalt an gelöstem und somit spontan freisetzbarem sowie der Gehalt an inhärentem, erst während einer Wärmebehandlung freisetzbare Gehalt an H₂S bestimmt werden. Darauf basierend lässt sich die für Einhaltung von Grenzwerten erforderliche Menge an H₂S-Scavenger unter verschiedenen Lagerbedingungen abschätzen.

## Patentansprüche

1. Verfahren zur Bestimmung des sich bei Warmlagerung von schwefelhaltigen Roh- und Rückstandsölen sowie schwefelhaltige Roh- und/oder Rückstandsöle enthaltenden Mineralöldestillaten einstellenden H₂S-Gehalts, in dem eine Probe des schwefelhaltigen Mineralöls in einem oberhalb 200 °C siedenden Lösemittel oder Lösemittelgemisch gelöst wird und die Lösung des schwefelhaltigen Mineralöls bei Temperaturen oberhalb 80 °C, vorzugsweise oberhalb 120°C, mit einem Trägergas durchströmt wird und die mit dem Trägergas ausgetragene Menge an Schwefelwasserstoff quantitativ analysiert wird, wobei das Durchströmen der Öllösung mit Trägergas so lange fortzusetzen ist, bis die Konzentration des vom Analysator detektierten H₂S auf den Basiswert zurückgegangen ist.

2. Verfahren zur Bestimmung der Konzentrationen von gelöstem Schwefelwasserstoff und inhärentem Schwefelwasserstoff in schwefelhaltigen Roh- und Rückstandsölen sowie schwefelhaltige Roh- und/oder Rückstandsöle enthaltenden Mineralöldestillaten, in dem eine Probe des schwefelhaltigen Mineralöls in einem oberhalb 200 °C siedenden Lösemittel oder Lösemittelgemisch gelöst wird und die Lösung des schwefelhaltigen Mineralöls zuerst zur Bestimmung des gelösten H₂S bei einer Temperatur unterhalb 100 °C mit einem Trägergas durchströmt und die mit dem Trägergas ausgetragene Menge an Schwefelwasserstoff quantitativ analysiert wird, wobei das Durchströmen der Öllösung mit Trägergas so lange fortzusetzen ist, bis die Konzentration des vom Analysator detektierten H₂S auf den Basiswert zurückgegangen ist und anschließend die derart behandelte Probe zur Bestimmung des inhärenten H₂S unter weiterem Spülen mit Trägergas auf Temperaturen oberhalb 100 °C erhitzt und das mit dem Trägergas ausgetragene H₂S quantitativ analysiert wird, und wobei die Temperatur zur Bestimmung des inhärenten H₂S mindestens 10 °C höher ist als die Temperatur zur Bestimmung des gelösten H₂S, wobei das Durchströmen der Öllösung mit Trägergas so lange fortzusetzen ist, bis die Konzentration des vom Analysator detektierten H₂S auf den Basiswert zurückgegangen ist.

3. Verfahren nach Anspruch 2, bei dem die Bestimmung des inhärenten H₂S bei Temperaturen oberhalb 120 °C erfolgt.

4. Verfahren nach einem oder mehreren der Ansprüche 1 bis 3, bei dem als Trägergas ein chemisch inertes Gas eingesetzt wird.

5. Verfahren nach einem oder mehreren der Ansprüche 1 bis 4, bei dem als Trägergas Stickstoff eingesetzt wird.

6. Verfahren nach einem oder mehreren der Ansprüche 1 bis 3, bei dem als Trägergas Sauerstoff oder eine Mischung aus Sauerstoff und einem oder mehreren chemisch inerten Gasen eingesetzt wird.

7. Verfahren nach Anspruch 6, bei dem als Trägergas Luft eingesetzt wird.

8. Verfahren nach Anspruch 6 oder 7, bei dem das im Trägergas ausgetragene SO₂ quantitativ bestimmt und nach Umrechnung auf H₂S diesem hinzugerechnet wird.

9. Verfahren nach einem oder mehreren der Ansprüche 1 bis 8, bei dem das Trägergas über eine Fritte in die Lösung des schwefelhaltigen Roh- und Rückstandsöls bzw. des diese enthaltenden Mineralöldestillats eingeleitet wird.

10. Verfahren nach einem oder mehreren der Ansprüche 1 bis 9, bei dem das Lösemittel oder Lösemittelgemisch vorwiegend aliphatisch ist.

11. Verfahren nach einem oder mehreren der Ansprüche 1 bis 10, bei dem das Lösemittel oder Lösemittelgemisch eine Jodzahl von weniger als 20 g J₂/100g aufweist.

12. Verfahren nach einem oder mehreren der Ansprüche 1 bis 11, bei dem das schwefelreiche Rückstandsöl Bitumen, Schweröl oder Bunkeröl ist.

13. Verfahren nach einem oder mehreren der Ansprüche 1 bis 12, bei dem das Mineralöldestillat eine hochsiedende Fraktion der Mineralöldestillation aus der Vakuumdestillation oder aus Crack- oder anderen Konversionsanlagen ist.

14. Verfahren zur Bestimmung der zum Binden von H₂S bei Lagerung von schwefelhaltigen Roh- und Rückstandsölen sowie schwefelhaltige Roh- und Rückstandsöle enthaltenden Mineralöldestillaten erforderlichen Konzentration an H₂S-Scavengern, in dem der Gehalt des schwefelhaltigen Mineralöls an gelöstem und inhärentem Schwefelwasserstoff gemäß einem oder mehreren der Ansprüche 1 bis 13 quantitativ analysiert und daraus die für dauerhafte Absenkung des H₂S-Gehalts erforderliche Menge an H₂S-Scavenger berechnet wird.

## Claims

1. A process for determining the H₂S content which is established in the course of high-temperature storage of sulfur-containing crude oils and residue oils and of mineral oil distillates comprising sulfur-containing crude oils and/or residue oils, in which a sample of the sulfur-containing mineral oil is dissolved in a solvent or solvent mixture which boils above 200°C and a carrier gas is conducted through the solution of the sulfur-containing mineral oil at temperatures above 80°C, preferably above 120°C, and the amount of hydrogen sulfide discharged with the carrier gas is quantitatively analyzed, wherein the flow of carrier gas through the oil solution should be continued until the concentration of the H₂S detected by the analyzer has returned to the base value.

2. A process for determining the concentrations of dissolved hydrogen sulfide and inherent hydrogen sulfide in sulfur-containing crude oils and residue oils and in mineral oil distillates comprising sulfur-containing crude oils and/or residue oils, in which a sample of the sulfur-containing mineral oil is dissolved in a solvent or solvent mixture which boils above 200°C and the dissolved H₂S is first determined by conducting a carrier gas through the solution of the sulfur-containing mineral oil at a temperature below 100°C and quantitatively analyzing the amount of hydrogen sulfide discharged with the carrier gas, wherein the flow of carrier gas through the oil solution should be continued until the concentration of the H₂S detected by the analyzer has returned to the base value, and then the inherent H₂S is determined by heating the sample treated in such a way to temperatures above 100°C while continuing to purge with carrier gas and quantitatively analyzing the H₂S discharged with the carrier gas, and the temperature for determination of the inherent H₂S being at least 10°C higher than the temperature for determination of the dissolved H₂S, wherein the flow of carrier gas through the oil solution should be continued until the concentration of the H₂S detected by the analyzer has returned to the base value.

3. The process as claimed in claim 2, in which the inherent H₂S is determined at temperatures above 120°C.

4. The process as claimed in one or more of claims 1 to 3, in which the carrier gas used is a chemically inert gas.

5. The process as claimed in one or more of claims 1 to 4, in which the carrier gas used is nitrogen.

6. The process as claimed in one or more of claims 1 to 3, in which the carrier gas used is oxygen or a mixture of oxygen and one or more chemically inert gases.

7. The process as claimed in claim 6, in which the carrier gas used is air.

8. The process as claimed in claim 6 or 7, in which the SO₂ discharged in the carrier gas is quantitatively determined, converted to H₂S and added on.

9. The process as claimed in one or more of claims 1 to 8, in which the carrier gas is introduced through a frit into the solution of the sulfur-containing crude oil and residue oil or of the mineral oil distillate comprising these.

10. The process as claimed in one or more of claims 1 to 9, in which the solvent or solvent mixture is predominantly aliphatic.

11. The process as claimed in one or more of claims 1 to 10, in which the solvent or solvent mixture has an iodine number of less than 20 g of J₂/100 g.

12. The process as claimed in one or more of claims 1 to 11, in which the sulfur-rich residue oil is bitumen, heavy oil or bunker oil.

13. The process as claimed in one or more of claims 1 to 12, in which the mineral oil distillate is a high-boiling fraction from mineral oil distillation arising from vacuum distillation or from cracking plants or other conversion plants.

14. A process for determining the concentration of H₂S scavengers required for binding of H₂S in the course of storage of sulfur-containing crude oils and residue oils and of mineral oil distillates comprising sulfur-containing crude oils and residue oils, in which the content in the sulfur-containing mineral oil of dissolved and inherent hydrogen sulfide is quantitatively analyzed according to one or more of claims 1 to 13 and the amount of H2S scavengers required for the permanent lowering of the H₂S content is calculated therefrom.

## Revendications

1. Procédé pour la détermination de la teneur en H₂S qui se règle lors du stockage à chaud de pétrole brut et d'huile résiduelle contenant du soufre ainsi que de distillats d'huile minérale contenant du pétrole brut et/ou de l'huile résiduelle contenant du soufre, dans lequel un échantillon de l'huile minérale contenant du soufre est dissous dans un solvant ou un mélange de solvants présentant un point d'ébullition supérieur à 200°C et la solution de l'huile minérale contenant du soufre est traversée par un gaz support à des températures supérieures à 80°C, de préférence supérieures à 120°C, et la quantité d'acide sulfhydrique évacuée par le gaz support est analysée quantitativement, le passage du gaz support à travers la solution d'huile devant être poursuivi jusqu'à ce que la concentration en H₂S détectée par l'analyseur soit revenue à la valeur de base.

2. Procédé pour la détermination des concentrations en acide sulfhydrique dissous et en acide sulfhydrique inhérent dans du pétrole brut et de l'huile résiduelle contenant du soufre ainsi que dans des distillats d'huile minérale contenant du pétrole brut et/ou de l'huile résiduelle contenant du soufre, dans lequel un échantillon de l'huile minérale contenant du soufre est dissous dans un solvant ou un mélange de solvants présentant un point d'ébullition supérieur à 200°C et la solution de l'huile minérale contenant du soufre, pour la détermination du H₂S dissous, est d'abord traversée par un gaz support à une température inférieure à 100°C et la quantité d'acide sulfhydrique évacuée par le gaz support est analysée quantitativement, le passage du gaz support à travers la solution d'huile devant être poursuivi jusqu'à ce que la concentration en H₂S détectée par l'analyseur soit revenue à la valeur de base, et l'échantillon ainsi traité, pour la détermination du H₂S inhérent, est ensuite chauffé à des températures supérieures à 100°C tout en poursuivant le rinçage par le gaz support et le H₂S évacué par le gaz support est déterminé quantitativement, la température pour la détermination du H₂S étant supérieure d'au moins 10°C à la température pour la détermination du H₂S dissous, le passage du gaz support à travers la solution d'huile devant être poursuivi jusqu'à ce que la concentration en H₂S détectée par l'analyseur soit revenue à la valeur de base.

3. Procédé selon la revendication 2, dans lequel la détermination du H₂S inhérent a lieu à des températures supérieures à 120°C.

4. Procédé selon l'une ou plusieurs des revendications 1 à 3, dans lequel on utilise, comme gaz support, un gaz chimiquement inerte.

5. Procédé selon l'une ou plusieurs des revendications 1 à 4, dans lequel on utilise de l'azote comme gaz support.

6. Procédé selon l'une ou plusieurs des revendications 1 à 3, dans lequel on utilise, comme gaz support, de l'oxygène ou un mélange d'oxygène et d'un ou de plusieurs gaz chimiquement inertes.

7. Procédé selon la revendication 6, dans lequel de l'air est utilisé comme gaz support.

8. Procédé selon la revendication 6 ou 7, dans lequel le SO₂ évacué dans le gaz support est déterminé quantitativement et ajouté après transformation par calcul en H₂S.

9. Procédé selon l'une ou plusieurs des revendications 1 à 8, dans lequel le gaz support est introduit via une fritte dans la solution de pétrole brut et d'huile résiduelle contenant du soufre ou du distillat d'huile minérale les contenant.

10. Procédé selon l'une ou plusieurs des revendications 1 à 9, dans lequel le solvant ou le mélange de solvants est principalement aliphatique.

11. Procédé selon l'une ou plusieurs des revendications 1 à 10, dans lequel le solvant ou le mélange de solvants présente un indice d'iode inférieur à 20 g d'I₂/100 g.

12. Procédé selon l'une ou plusieurs des revendications 1 à 11, dans lequel l'huile résiduelle riche en soufre est le bitume, le fuel lourd ou le fuel de soute.

13. Procédé selon l'une ou plusieurs des revendications 1 à 12, dans lequel le distillat d'huile minérale est une fraction à point d'ébullition élevé de la distillation d'huile minérale provenant de la distillation sous vide ou d'installations de craquage ou d'autres installations de conversion.

14. Procédé pour la détermination de la concentration nécessaire en pièges de H₂S pour fixer le H₂S lors du stockage de pétrole brut et d'huile résiduelle contenant du soufre ainsi que des distillats d'huile minérale contenant du pétrole brut et de l'huile résiduelle contenant du soufre, dans lequel la teneur de l'huile minérale contenant du soufre en acide sulfhydrique dissous et inhérent est analysée quantitativement selon l'une ou plusieurs des revendications 1 à 13 et la quantité en piège de H₂S nécessaire pour la diminution durable de la teneur en H₂S est calculée à partir de celle-ci.
